# EUROPÄISCHE PATENTSCHRIFT

(11) **EP 1 399 118 B1**
(45) Veröffentlichungstag und Bekanntmachung des Hinweises auf die Patenterteilung: **23.07.2008**
(21) Anmeldenummer: 01997289.2
(22) Anmeldetag: 21.11.2001
(51) Int. Cl.: A61K 8/44, A61K 8/73, A61K 8/81, A61Q 9/02

(54) **Verwendung von kosmetischen Zubereitungen für die Rasur mittels eines Elektrorasiergerätes**
Use of cosmetic preparations for shaving with an electric device
Utilisations des préparations cosmétiques destinées au rasage au moyen d'un rasoir éléctrique

(30) Priorität: 22.11.2000 DE 10057925
(43) Veröffentlichungstag der Anmeldung: 24.03.2004
(73) Patentinhaber: Beiersdorf AG, 20253 Hamburg (DE); Koninklijke Philips Electronics N.V., 5621 BA Eindhoven (NL)
(72) Erfinder: HEIKE, Kerstin, 22453 Hamburg (DE); KADEN, Waltraud, 25469 Halstenbek (DE); GOHLA, Sven, 21077 Hamburg (DE)
(86) Internationale Anmeldenummer: PCT/EP2001/013512
(87) Internationale Veröffentlichungsnummer: WO 2002/041860

(56) Entgegenhaltungen:
- DE-A- 19 646 233

## Beschreibung

Die Erfindung betrifft kosmetische Zubereitungen, insbesondere Rasiergele, welche zur Rasur mittels eines elektrischen Rasiergerätes, geeignet sind. Insbesondere betrifft die Erfindung solche Rasiergele, welche dazu bestimmt sind, vor oder während der Elektrorasur auf die Haut aufgebracht zu werden.

Der Wuchs des Barthaares wird beim heranwachsenden Manne durch die gesteigerte Bildung männlicher Hormone während der Pubertät ausgelöst. Hormonelle Störungen bei der Frau können ebenfalls zu einer Form des Bartwuchses führen, der allerdings, wiewohl durchaus unangenehm, in aller Regel in seiner Ausprägung deutlich hinter dem des männlichen Bartwuchses zurückbleibt

Die Rasur kann durch mancherlei Zwänge - z.B. religiöser oder kultureller Art - motiviert (auch tabuisiert) werden, im einfachsten Falle ist Bartwuchs für die betreffende Person schlichtweg aus kosmetischen Gründen unerwünscht.

Die Rasur wird entweder trocken oder naß durchgeführt. Wo bei der Naßrasur chemische Hilfsmittel unerläßlich sind, sind diese bei der Trockenrasur doch wenigstens empfehlenswert um eine möglichst dichte Rasur zu bewirken, d.h., das Barthaar möglichst nahe an der Hautoberfläche abzuschneiden.

Im Rahmen der vorliegenden Offenbarung werden zur Vereinfachung des Sprachgebrauches stets der Begriff "Elektrorasierer" und Synonyma verwendet werden, wenn von elektrischen Rasiergeräten die Rede ist, da solche - neben Naßrasierklingen - in der Praxis die einzigen bedeutenden Rasiergeräte darstellen.

Bei der Elektrorasur, die auch als "Trockenrasur bezeichnet wird, muß, im Gegensatze zur "Naßrasur" das Barthaar steif sein, um von den Scherköpfen der Elektrorasieren erfaßt werden zu können. Dies bedeutete in der Vergangenheit, es mußte auch trocken und dadurch spröde sein. Zu diesem Zwecke wurden sogenannte "Pre-shaves" (auch: "Elektro-shaves") aufgetragen, meist hochalkoholische Präparate (typischerweise um 80 Gew.-% an Ethanol) durch deren Verdunsten auch Feuchtigkeit vom Barthaar mitgeschleppt wurde.

Nachteil der bekannten Rasierzubereitungen ist, daß sie nur ungenügende Gleitfähigkeit zwischen dem Scherkopf und der Hautoberfläche erzeugen. Dies ist insbesondere dann der Fall, wenn hochalkoholische Präparate angewandt werden, die eine wenig gleitfähige Sebumschicht auf der Hautoberfläche zurücklassen.

Ferner ist ein Übelstand des Standes der Technik, daß durch die herkömmlichen Zubereitungen eine gründliche Rasur nur eingeschränkt bewerkstelligt werden kann, daß die elektrischen Kontakte des Elektrorasierers durch Bestandteile der Rezepturen beeinträchtigt, beschädigt oder sogar zerstört werden können, daß auch sonstige Metallteile der Korrosion ausgesetzt sind und/oder daß die in der Haarauffangkammer des Elektrorasierers anfallenden Bartstoppel, vermengt mit der Rasierzubereitung, zu auch durch Wasser schwer entfernbaren Gemischen führt Letztere bergen zudem das Risiko, Mikroorganismen als Nährboden zu dienen.

In der WO98/08659 wird ein Rasiersystem beschrieben, welches einen Elektrorasierer und einen Behälter umfaßt. Der Rasierer umfaßt einen Scherkopf und eine Haarauffangkammer, welche den Behälter aufnehmen kann. Der Behälter umfaßt ein Reservoir, welches mit einer Rasierzubereitung gefüllt ist, wobei die Rasierzubereitung durch entsprechende Vorrichtungen im Scherkopf auf die Haut aufgetragen werden kann.

Rasierzubereitungen in flüssiger Form für solche Zwecke sind an sich bekannt. Üblicherweise stellen solche flüssige oder halbflüssige bis pastöse wäßrige, gelförmige oder in Form von Emulsionen vorliegende Zubereitungen dar,

Die WO98/4747 beschreibt beispielsweise eine Rasierflüssigkeit für ein Rasiersystem, welches Wasser als Hauptbestandteil umfaßt, femer übliche Zusatzstoffe und ein oberflächenaktives Agens, wobei die Rasierflüssigkeit eine Emulsion wenigstens eines flüssigen Kohlenwasserstoffes in Wasser darstellt und das oberflächenaktive Agens ein C₆-C₁₈-Alkylglucosid ist.

Ein weiterer Nachteil ist, daß die Zubereitungen während des Auftrags oder während der Rasur Schaum entwickeln, da dieser die Rasur mit einem elektrischen Rasiergerät in nicht unerheblichem Ausmaße stört.

Noch ein weiterer Nachteil ist, daß die Elektrorasur die Gesichtshaut mehr oder weniger stark reizt. Rasierzubereitungen sollten also ihrerseits nicht in nennenswertem Maße Reizpotential hinzufügen.

Übliche, und sich gerade in neuerer Zeit immer weiter verbreitende kosmetische und dermatologische Zubereitungsformen sind Gele.

Im technischen Sinne werden unter Gelen verstanden: Relativ formbeständige, leicht verformbare disperse Systeme aus zumindest zwei Komponenten, welche in der Regel aus einem - meist festen - kolloid zerteilten Stoff aus langkettigen Molekülgruppierungen (z.B. Gelatine, Kieselsäure, Polysaccharide) als Gerüstbildner und einem flüssigen Dispersionsmittel (z.B. Wasser) bestehen. Der kolloidal zerteilte Stoff wird oft als Verdickungs- oder Geliermittel bezeichnet Er bildet ein räumliches Netzwerk im Dispersionsmittel, wobei einzelne kolloidal vorliegende Partikel über elektrostatische Wechselwirkung miteinander mehr oder weniger fest verknüpft sein können. Das Dispersionsmittel, welches das Netzwerk umgibt, zeichnet sich durch elektrostatische Affinität zum Geliermittel aus, d.h., ein vorwiegend polares (insbesondere: hydrophiles) Geliermittel geliert vorzugsweise ein polares Dispersionsmittel (insbesondere: Wasser), wohingegen ein vorwiegend unpolares Geliermittel vorzugsweise unpolare Dispersionsmittel geliert.

Starke elektrostatische Wechselwirkungen, welche beispielsweise in Wasserstoffbrückenbindungen zwischen Geliermittel und Dispersionsmittel, aber auch zwischen Dispersionsmittelmolekülen untereinander verwirklicht sind, können zu starker Vernetzung auch des Dispersionsmittels führen. Hydrogele können zu fast 100 % aus Wasser bestehen (neben beispielsweise ca. 0,2 - 1,0 % eines Geliermittels) und dabei durchaus feste Konsistenz besitzen. Der Wasseranteil liegt dabei in eisähnlichen Strukturelementen vor, so daß Gele daher ihrer Namensherkunft [aus lat. "gelatum" = "Gefrorenes" über den alchimistischen Ausdruck "gelatina" (16. Jhdt.) für nhdt. "Gelatine"] durchaus gerecht werden.

In der kosmetischen und pharmazeutischen Galenik sind femer auch Lipogele und Oleogele (aus Wachsen, Fetten und fetten Ölen) sowie Carbogele (aus Paraffin oder Petrolatum) geläufig. In der Praxis unterscheidet man Oleogele, welche praktisch wasserfrei vorliegen, Hydrogele, welche praktisch fettfrei sind. Meistens sind Gele durchsichtig. In der kosmetischen bzw. pharmazeutischen Galenik zeichnen sich Gele in aller Regel durch halbfeste, oft fließfähige Konsistenz aus.

Ferner sind sogenannte Tensidgele gebräuchliche Zubereitungen des Standes der Technik. Darunter versteht man Systeme, die neben Wasser eine hohe Konzentration an Emulgatoren aufweisen, typischerweise mehr als ca. 25 Gew.-%, bezogen auf die Gesamtzusammensetzung. Solubilisiert man in diese Tensidgele, fachsprachlich auch "surfactant gels" genannt, Ölkomponenten, werden Mikroemulsionsgele erhalten, welche auch als "ringing gels" bezeichnet werden. Durch Zusatz von nichtionischen Emulgatoren, beispielsweise Alkylpolyglycosiden, lassen sich kosmetisch elegantere Mikroemulsionsgele erhalten.

DE-A-196 46 233 offenbart Rasiergelzusammensetzungen, die Carboxylvinylpolymer, Polysaccharid, N-Lauroylsarcosinat und Wasser enthalten.

Obwohl Gele landläufig als besonders hautfreundlich gelten, ist jedoch auch ihnen in der Regel ein relativ hoher Gehalt an Substanzen eigen, welche wenigstens einige der eingangs geschilderten Übelstände in mehr oder weniger ausgeprägtem Umfange auftreten lassen.

Es wurde nun gefunden, daß kosmetische Zubereitungen, insbesondere Rasiergele, welche zur Rasur mittels elektrischen Rasiergerätes, geeignet sind, gekennzeichnet durch einen Gehalt an
(1) einem oder mehreren N-Acylsarkosinaten
(2) einem oder mehreren Polysacchariden
(3) einer oder mehreren Substanzen, welche in Wasser ein Gelgerüst bilden, welche keine Polysaccharide darstellen und welche vorteilhafterweise grenzflächenaktive Eigenschaften aufweisen
(4) einem oder mehreren Lösungsvermittlern, bevorzugt gewählt aus der Gruppe der polyethoxylierten gesättigten Fett- oder Ölkomponenten
(5) Wasser
die Nachteile des Standes der Technik beseitigen.

Die erfindungsgemäßen Zubereitungen haben ein außergewöhnlich niedriges Hautreizungspotential. In erstaunlicher Weise erzeugen sie hohe Gleitfähigkeit zwischen dem Scherkopf und der Hautoberfläche und gewährleisten eine verbesserte Rasur, ohne daß die Metallteile eines zu verwendenden Elektrorasierers Gefahr läuft, korrodierendem Einfluß ausgesetzt zu werden. Bei erfindungsgemäßer Verwendung ist der Scherbereich des Elektrorasierers leicht mit Wasser, auch ohne weitere Reinigungszusätze, zu reinigen, ohne daß lästige Rückstände verblieben. Insbesondere vorteilhaft ist die Verwendung der erfindungsgemäßen Zubereitung bei der Rasur mit Rasiersystemen gemäß oder analog der WO98/08659.

N-Acylsarkosinate sind Tenside, die im Prinzip als "unterbrochene Seifen" betrachtet werden können, weil der einzige Unterschied zwischen einer Seife (Fettsäure) und dem Sarkosinat darin besteht, daß die Fettsäure durch den Einbau einer Methylamidogruppe unterbrochen wird:

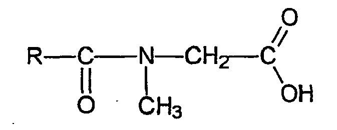

Für R stehen bevorzugt Lauroyl-, Kokoyl-, Myristoyl-, Oleyl-, Stearyl- oder ein Gemisch dieser Reste. Besonders bevorzugt ist das Natriumlauroylsarkosinat (CAS-Nr. 137-16-6), Es wird als 30 %ige wäßrige Lösung angeboten (beispielsweise unter den Handelsbezeichnungen Sarkosyl® NL-30, Maprosyl® 30 und Rokosyl® NL 30).

Erfindungsgemäß enthalten die Zubereitungen vorteilhaft 0,01 bis 10 Gew.-%, besonders vorteilhaft 0,01 bis 5 Gew.-%, ganz besonders bevorzugt 0,5 bis 3 Gew.-% an einem oder mehreren N-Acylsarkosinaten, jeweils bezogen auf das Gesamtgewicht der Zubereitungen.

Es ist von Vorteil wasserlösliche und/oder in Wasser quellbare und/oder mit Hilfe von Wasser gelierbare Polysaccharide zu verwenden.

Vorteilhaftes Polysaccharid im Sinne der vorliegenden Erfindung ist beispielsweise Carrageen, ein gelbildender und ähnlich wie Agar aufgebauter Extrakt aus nordatlant, zu den Florideen zählenden Rotalgen (Chondrus crispus und Gigartina stellata).

Häufig wird die Bezeichnung Carrageen für das getrocknete Algenprodukt und Carrageenan für den Extrakt aus diesem verwendet. Das aus dem Heißwasserextrakt der Algen ausgefällte Carrageen ist ein farbloses bis sandfarbenes Pulver mit einem Molekulargewichtsbereich von 100 000-800 000 und einem Sulfat-Gehalt von ca. 25 %. Carrageen, das in warmem Wasser sehr leicht lösl. ist; beim Abkühlen bildet sich ein thixotropes Gel, selbst wenn der Wassergehalt 95-98 % beträgt. Die Festigkeit des Gels wird durch die Doppelhelix-Struktur des Carrageens bewirkt. Beim Carrageenan unterscheidet man drei Hauptbestandteile: Die gelbildende κ-Fraktion besteht aus D-Galactose-4-sulfat und 3,6-Anhydro-α-D-galactose, die abwechselnd in 1,3- und 1,4-Stellung glykosidisch verbunden sind (Agar enthält demgegenüber 3,6-Anhydro-α-L-galactose). Die nicht gelierende λ-Fraktion ist aus 1,3-glykosidisch verknüpften D-Galactose-2-sulfat und 1,4-verbundenen D-Galactose-2,6-disulfat-Resten zusammengesetzt u. in kaltem Wasser leicht löslich. Das aus D-Galactose-4-sulfat in 1,3-Bindung und 3,6-Anhydro-α-D-galactose-2-sulfat in 1,4-Bindung aufgebaute τ-Carrageenan ist sowohl wasserlöslich als auch gelbildend. Weitere Carrageen-Typen werden ebenfalls mit griechischen Buchstaben bezeichnet: α, β, γ, µ, ν, ξ, π, ω, χ. Auch die Art vorhandener Kationen (K⁺, NH₄⁺, Na⁺, Mg²⁺, Ca²⁺) beeinflußt die Löslichkeit der Carrageene.

Von besonderem Vorteil können femer verwendet werden Hyaluronsäure, Chitosan sowie auch andere wasserlösliche und/oder in Wasser quellbare und/oder mit Hilfe von Wasser gelierbare Polysaccharide zu verwenden.

Hyaluronsäure zeichnet sich durch die Struktur 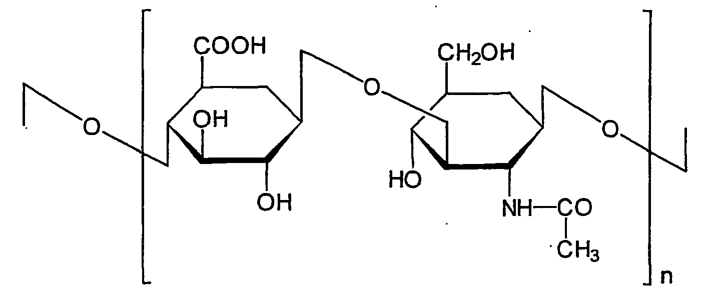 aus.

Wenn Hyaluronsäure das oder eines der verwendeten Polysaccharide darstellt, ist es von Vorteil, solche mit Molekulargewichten zwischen 30.000 und 8.000.000 zu wählen, insbesondere solche mit Molekulargewichten zwischen 500.000 und 1.500.000.

Chitosan ist gekennzeichnet durch folgende Strukturformel:

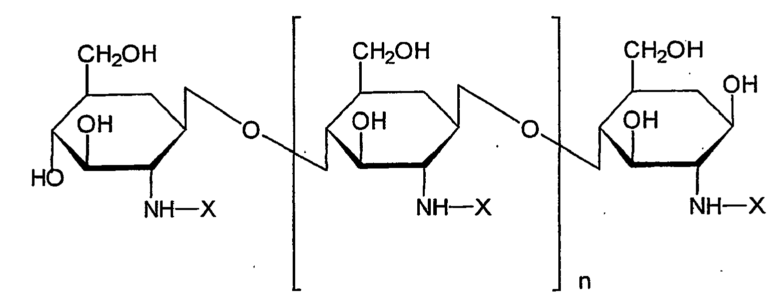

Dabei nimmt n Werte bis zu ca. 2.000 an, X stellt entweder den Acetylrest oder Wasserstoff dar. Chitosan entsteht durch Deacetylierung und teilweise Depolymerisation (Hydrolyse) von Chitin, welches durch die Strukturformel 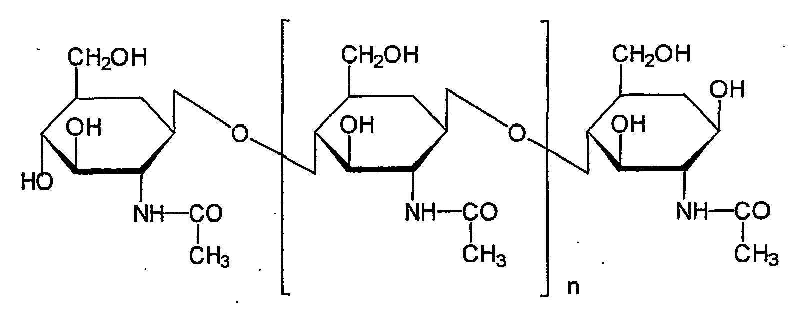 gekennzeichnet ist Chitin ist wesentlicher Bestandteil des Ektoskeletts ['o χ 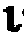 των = grch.: der Panzerrock] der Gliederfüßer (z.B. Insekten, Krebse, Spinnen) und wird auch in Stützgeweben anderer Organismen (z.B. Weichtiere, Algen, Pilze) gefunden.

Chitosan ist ein in der Haarpflege bekannter Rohstoff. Es eignet sich, besser als das ihm zugrundeliegende Chitin, als Verdicker oder Stabilisator und verbessert die Adhäsion und Wasserresistenz von polymeren Filmen. Stellvertretend für eine Vielzahl von Fundstellen des Standes der Technik: H. P. Fiedler, "Lexikon der Hilfsstoffe für Pharmazie, Kosmetik und angrenzende Gebiete", dritte Auflage 1989, Editio Cantor, Aulendorf, S. 293, Stichwort "Chitosan".

Erfindungsgemäß bevorzugt sind Chitosane mit einem Deacetylierungsgrad > 60 %, insbesondere > 80%. Unter diesen besonders bevorzugt sind solche, deren 1%-ige wäßrige Lösung eine Viskosität von 4.500 - 5.500 mPas (Brookfield, Spindel 5, 10 UpM), insbesondere 5.000 mPas aufweist.

Wenn Chitosan das oder eines der verwendeten Polysaccharide darstellt, ist es von Vorteil, solches mit Molekulargewichten zwischen 3.000 und 2.000.000 zu wählen, insbesondere solches mit Molekulargewichten zwischen 10.000 und 500.000.

Bevorzugtes Polysaccharid ist das Xanthangummi (CAS-Nr. 11138-66-2), welches ein anionisches Heteropolysaccharid ist, das in der Regel durch Fermentation aus Maiszucker gebildet und als Kaliumsalz isoliert wird. Es wird von Xanthomonas campestris und einigen anderen Species unter aeroben Bedingungen mit einem Molekulargewicht von 2×10⁶ bis 24×10⁶ produziert. Xanthan wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Xanthan ist die Bezeichnung für das erste mikrobielle anionische Heteropolysaccharid. Es wird von Xanthomonas campestris und einigen anderen Species unter aeroben Bedingungen mit einem Molekulargewicht von 2-15 10⁶ produziert. Xanthan wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Die Anzahl der Pyruvat-Einheiten bestimmt die Viskosität des Xanthans. Xanthan wird in zweitägigen Batch-Kulturen mit einer Ausbeute von 70-90 %, bezogen auf eingesetztes Kohlenhydrat, produziert. Dabei werden Ausbeuten von 25-30 g/l erreicht. Die Aufarbeitung erfolgt nach Abtöten der Kultur durch Fällung mit z. B. 2-Propanol. Xanthan wird anschließend getrocknet und gemahlen. Xanthan ist gekennzeichnet durch die Struktur 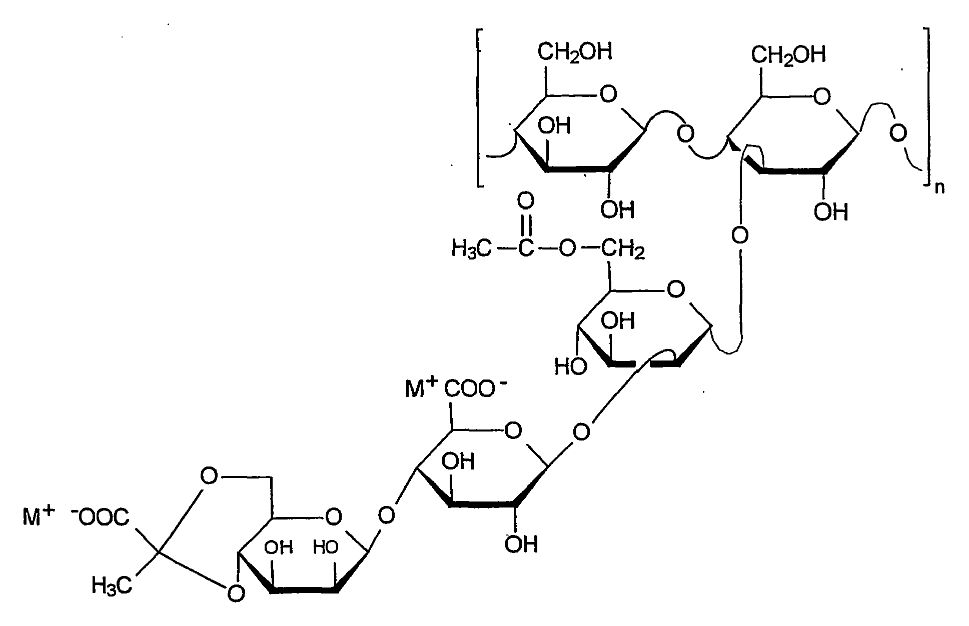 wobei M⁺ Na⁺, K⁺ oder ein halbes Ca²⁺-Äquivalent bedeuten können.

Erfindungsgemäß enthalten die Zubereitungen vorteilhaft 0,01 bis 10 Gew.-%, besonders vorteilhaft 0,01 bis 5 Gew.-%, ganz besonders bevorzugt 0,5 bis 3 Gew.-% an einem oder mehreren Polysacchariden, jeweils bezogen auf das Gesamtgewicht der Zubereitungen.

Die Substanz oder Substanzen, welche in Wasser ein Gelgerüst bilden, welche keine Polysaccharide darstellen und welche vorteilhafterweise grenzflächenaktive Eigenschaften aufweisen, wird oder werden vorzugsweise gewählt aus der Gruppe der Polyacrylate. Erfindungsgemäß vorteilhafte Polyacrylate sind Acrylat-Alkylacrylat-Copolymere, insbesondere solche, die aus der Gruppe der sogenannten Carbomere oder Carbopole (Carbopol® ist eigentlich eine eingetragene Marke der B. F. Goodrich Company) gewählt wer den. Insbesondere zeichnen sich das oder die erfindungsgemäß vorteilhaften Acrylat-Alkylacrylat-Copolymere durch die folgende Struktur aus:

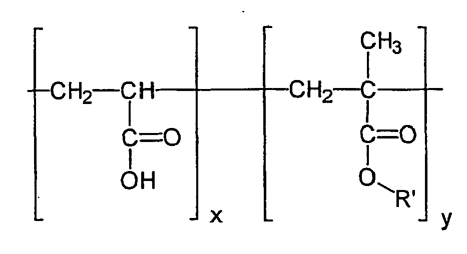

Darin stellen R' einen langkettigen Alkylrest und x und y Zahlen dar, welche den jeweiligen stöchiometrischen Anteil der jeweiligen Comonomere symbolisieren.

Ferner vorteilhaft sind Copolymere aus C₁₀₋₃₀-Alkylacrylaten und einem oder mehreren Monomeren der Acrylsäure, der Methacrylsäure oder deren Ester, die kreuzvemetzt sind mit einem Allylether der Saccharose oder einem Allylether des Pentaerythrit.

Erfindungsgemäß besonders vorteilhaft sind folgende Polyacrylattypen:

| | |
|---|---|
| Carbomer 907 | (Molgew. 450 000), |
| Carbomer 910 | (Molgew. 750 000), |
| Carbomer 941 und | |
| Carbomer 951 | (Molgew. 1 250 000), |
| Carbomer 934, | |
| Carbomer 940 und | |
| Carbomer 954 | (Molgew. 3 000 000), |
| Carbomer 940 | (Molgew. 4 000 000), |
| | |
| Carbomer 980, | (Molgew. 4 000 000), |
| Carbomer 981 | (Molgew. 1 250 000), |
| Carbomer 984 | (Molgew. 3 000 000), |

(Carbomer 980, 981 und 984 sind Acrylsäure-Polymerisate, die in einem Gemisch aus Ethylacetat und Cyclohexan polymerisiert werden)

| | |
|---|---|
| Carbomer 974 P | (Molgew. 3 000 000), |
| Carbomer 1342 | (Molgew. 1 300 000). |

Carbomer 1342 weist eine ähnliche Zusammensetzung auf wie die Pemulen-Typen. Pemulen ist die Handelsbezeichnung für ein Copolymeres aus C₁₀₋₃₀-Alkylacrylaten und einem oder mehreren Monomeren der Acrylsäure, der Methacrylsäure oder deren Ester, die kreuzvemetzt sind mit einem Allylether der Saccharose oder einem Allylether des Pentaerythrit (CTFA-Bezeichnung: Acrylates/C₁₀₋₃₀ Alkyl Acrylate Crosspolymer). Bei den Pemulenen handelt es sich um hochmolekulare Polymere mit einem hohen hydrophilen und zugleich mit einem geringen Gehalt an lipophilen Anteilen.

Carbomer 1342 ist die erfindungsgemäß bevorzugte Substanz, welche in Wasser ein Gelgerüst bildet.

Erfindungsgemäß enthalten die Zubereitungen vorteilhaft 0,01 bis 10 Gew.-%, besonders vorteilhaft 0,01 bis 5 Gew.-%, ganz besonders bevorzugt 0,5 bis 3 Gew.-% an einer oder mehreren Substanz oder Substanzen, welche in Wasser ein Gelgerüst bilden, welche keine Polysaccharide darstellen, jeweils bezogen auf das Gesamtgewicht der Zubereitungen.

Der oder die Lösungsvermittler wird vorteilhaft gewählt aus der Gruppe der polyethoxylierten gesättigten Fett- oder Ölkomponenten, insbesondere aus der Gruppe PEG-X-hydriertes Ricinusöl, wobei X vorteilhaft die Zahlen 40, 100 oder 200 annehmen kann.

Erfindungsgemäß enthalten die Zubereitungen vorteilhaft 0,01 bis 10 Gew.-%, besonders vorteilhaft 0,01 bis 5 Gew.-%, ganz besonders bevorzugt 0,5 bis 3 Gew.-% an einem oder mehreren Lösungsvermittlern, jeweils bezogen auf das Gesamtgewicht der Zubereitungen.

Der Wassergehalt der erfindungsgemäßen Zubereitungen beträgt in der Regel zwischen 70 und 98 Gew.-%, bezogen auf das Gesamtgewicht der Zubereitungen, insbesondere zwischen 85 und 95 Gew.-%

Es ist erfindungsgemäß vorteilhaft, den Zubereitungen weitere Verdicker zuzufügen, insbesondere beispielsweise Methylcellulosen, als welche die Methylether der Cellulose bezeichnet werden. Sie zeichnen sich durch die folgende Strukturformel aus 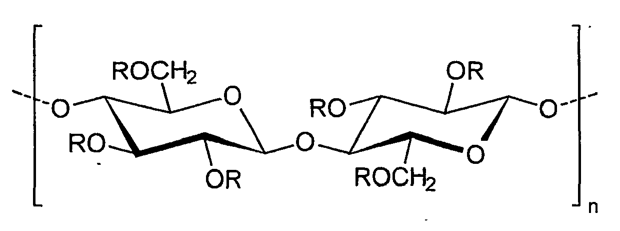 in der R ein Wasserstoff oder eine Methylgruppe darstellen kann.

Insbesondere vorteilhaft im Sinne der vorliegenden Erfindung sind die im allgemeinen ebenfalls als Methylcellulosen bezeichneten Cellulosemischether, die neben einem dominierenden Gehalt an Methyl- zusätzlich 2-Hydroxyethyl-, 2-Hydroxypropyl- oder 2-Hydroxybutyl-Gruppen enthalten. Besonders bevorzugt sind (Hydroxypropyl)methylcellulosen, beispielsweise die unter der Handelsbezeichnung Methocel E4M bei der Dow Chemical Comp. erhältlichen.

Erfindungsgemäß femer vorteilhaft ist Natriumcarboxymethylcellulose, das Natrium-Salz des Glykolsäureethers der Cellulose, für welches R in Strukturformel I ein Wasserstoff und/oder CH₂-COONa darstellen kann. Besonders bevorzugt ist die unter der Handelsbezeichnung Natrosol Plus 330 CS bei Aqualon erhältliche, auch als Cellulose Gum bezeichnete Natriumcarboxymethylcellulose.

Es kann gegebenenfalls erfindungsgemäß von Vorteil sein, den Zubereitungen außer den Sarkosinaten ein oder mehrere weitere Tenside zuzusetzen. Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekülteil, für eine Herabsetzung der Oberflächenspannung des Wassers, die Benetzung der Haut, die Erleichterung der Schmutzentfemung und -lösung, ein leichtes Abspülen und - je nach Wunsch - für Schaumregulierung.

Bei den hydrophilen Anteilen eines Tensidmoleküls handelt es sich meist um polare funktionelle Gruppen, beispielweise -COO⁻, -OSO₃²⁻, -SO₃⁻, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:
● anionische Tenside,
● kationische Tenside,
● amphotere Tenside und
● nichtionische Tenside.

Anionische Tenside weisen als funktionelle Gruppen in der Regel Carboxylat-, Sulfat- oder Sulfonatgruppen auf. In wäßriger Lösung bilden sie im sauren oder neutralen Milieu negativ geladene organische Ionen. Kationische Tenside sind beinahe ausschließlich durch das Vorhandensein einer quarternären Ammoniumgruppe gekennzeichnet. In wäßriger Lösung bilden sie im sauren oder neutralen Milieu positiv geladene organische Ionen. Amphotere Tenside enthalten sowohl anionische als auch kationische Gruppen und verhalten sich demnach in wäßriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

| | | |
|---|---|---|
| RNH₂⁺CH₂CH₂COOH X⁻ | (bei pH=2) | X⁻ = beliebiges Anion, z.B. Cl⁻ |
| RNH₂⁺CH₂CH₂COO⁻ | (bei pH=7) | |
| RNHCH₂CH₂COO⁻ B⁺ | (bei pH=12) | B⁺ = beliebiges Kation, z.B. Na⁺ |

Typisch für nicht-ionische Tenside sind Polyether-Ketten. Nicht-ionische Tenside bilden in wäßrigem Medium keine Ionen.

### A. Anionische Tenside

Vorteilhaft zu verwendende anionische Tenside sind
Acylaminosäuren (und deren Salze), wie
1. Acylglutamate, beispielsweise Natriumacylglutamat, Di-TEA-palmitoylaspartat und Natrium Caprylic/ Capric Glutamat,
2. Acylpeptide, beispielsweise Palmitoyl-hydrolysiertes Milchprotein, Natrium Cocoyl-hydrolysiertes Soja Protein und Natrium-/ Kalium-Cocoyl-hydrolysiertes Kollagen,
3. Taurate, beispielsweise Natriumlauroyltaurat und Natriummethylcocoyltaurat,
4. Acyllactylate, Lauroyllactylat, Caproyllactylat
5. Alaninate
Carbonsäuren und Derivate, wie
1. Carbonsäuren, beispielsweise Laurinsäure, Aluminiumstearat, Magnesiumalkanolat und Zinkundecylenat,
2. Ester-Carbonsäuren, beispielsweise Calciumstearoyllactylat, Laureth-6-Citrat und Natrium PEG-4-Lauramidcarboxylat,
3. Ether-Carbonsäuren, beispielsweise Natriumlaureth-13-Carboxylat und Natrium PEG-6-Cocamide Carboxylat,

Phosphorsäureester und Salze, wie beispielsweise DEA-Oleth-10-Rhosphat und Dilaureth-4 Phosphat,
Sulfonsäuren und Salze, wie
1. Acyl-isethionate, z.B. Natrium-/ Ammoniumcocoyl-isethionat,
2. Alkylarylsulfonate,
3. Alkylsulfonate, beispielsweise Natriumcocosmonoglyceridsulfat, Natrium C₂₋₁₄ Olefin-sulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat und Dinatriumundecylenamido-MEA-Sulfosuccinat
sowie
Schwefelsäureester, wie
1. Alkylethersulfat, beispielsweise Natrium-, Ammonium-, Magnesium-, MIPA-, TIPA-Laurethsulfat, Natriummyrethsulfat und Natrium C₁₂₋₁₃-Parethsulfat,
2. Alkylsulfate, beispielsweise Natrium-, Ammonium- und TEA-Laurylsulfat.

### B. Kationische Tenside

Vorteilhaft zu verwendende kationische Tenside sind
1. Alkylamine,
2. Alkylimidazole,
3. Ethoxylierte Amine und
4. Quaternäre Tenside.
5. Esterquats

Quaternäre Tenside enthalten mindestens ein N-Atom, das mit 4 Alkyl- und/oder Arylgruppen kovalent verbunden ist. Dies führt, unabhängig vom pH Wert, zu einer positiven Ladung. Vorteilhafte quaternäre Tenside sind Alkylbetain, Alkylamidopropylbetain und Alkyl-amidopropylhydroxysulfain. Kationische Tenside können femer bevorzugt im Sinne der vorliegenden Erfindung gewählt werden aus der Gruppe der quaternären Ammoniumverbindungen, insbesondere Benzyltrialkylammoniumchloride oder -bromide, wie beispielsweise Benzyldimethylstearylammoniumchlorid, femer Alkyltrialkylammoniumsalze, beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

### C. Amphotere Tenside

Vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.

### D. Nicht-ionische Tenside

Vorteilhaft zu verwendende nicht-ionische Tenside sind
1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Ether
7 Polyglycerinester, Diglycerinester, Monoglycerinester
8. Methylglucosester, Ester von Hydroxysäuren

Die erfindungsgemäßen kosmetischen Zubereitungen können kosmetische Hilfsstoffe enthalten, wie sie üblicherweise in solchen Zubereitungen verwendet werden, z.B. Konservierungsmittel, Bakterizide, Antioxidantien, Parfüme, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchthaltende Substanzen, Fette, Öle, Wachse oder andere übliche Bestandteile einer kosmetischen Formulierung wie Alkohole, Polyole, Polymere, Schaumstabilisatoren, Elektrolyte, pH-regulierende Stoffe, organische Lösungsmittel oder Silikonderivate.

Es ist erfindungsgemäß femer gegebenenfalls vorteilhaft, den Zubereitungen Komplexbildner zuzufügen.

Komplexbildner sind an sich bekannte Hilfsstoffe der Kosmetologie bzw. der medizinischen Galenik. Durch die Komplexierung von störenden Metallen wie Mn, Fe, Cu und anderer können beispielsweise unerwünschte chemische Reaktionen in kosmetischen oder dermatologischen Zubereitungen verhindert werden.

Komplexbildner, insbesondere Chelatoren, bilden mit Metallatomen Komplexe, welche bei Vorliegen eines oder mehrerer mehrbasiger Komplexbildner, also Chelatoren, Metallacyclen darstellen. Chelate stellen Verbindungen dar, in denen ein einzelner Ligand mehr als eine Koordinationsstelle an einem Zentralatom besetzt. In diesem Falle werden also normalerweise gestreckte Verbindungen durch Komplexbildung über ein Metall-Atom od. -Ion zu Ringen geschlossen. Die Zahl der gebundenen Liganden hängt von der Koordinationszahl des zentralen Metalls ab. Voraussetzung für die Chelatbildung ist, daß die mit dem Metall reagierende Verbindung zwei oder mehr Atomgruppierungen enthält, die als Elektronendonatoren wirken.

Der oder die Komplexbildner können vorteilhaft aus der Gruppe der üblichen Verbindungen gewählt werden, wobei bevorzugt mindestens eine Substanz aus der Gruppe bestehend aus Weinsäure und deren Anionen, Citronensäure und deren Anionen, Aminopolycarbonsäuren und deren Anionen (wie beispielsweise Ethylendiamintetraessigsäure (ED-TA) und deren Anionen, Nitrilotriessigsäure (NTA) und deren Anionen, Hydroxyethylendiaminotriessigsäure (HOEDTA) und deren Anionen, Diethylenaminopentaessigsäure (DPTA) und deren Anionen, trans-1,2-Diaminocyclohexantetraessigsäure (CDTA) und deren Anionen).

Der oder die Komplexbildner sind erfindungsgemäß vorteilhaft in kosmetischen oder dermatologischen Zubereitungen bevorzugt zu 0,01 Gew.-% bis 10 Gew.-%, bevorzugt zu 0,05 Gew.-% bis 5 Gew.-%, insbesondere bevorzugt zu 0,1 - 2,0 Gew.-%, bezogen auf das Gesamtgewicht der Zubereitungen, enthalten.

Die folgenden Beispiele sollen den Gegenstand der Erfindung näher erläutern, ohne ihn darauf einzuschränken.

| **Beispiele** | | | |
|---|---|---|---|
| | 1 | 2 | 3 |
| | Gew.-% | Gew.-% | Gew.-% |
| Decylglucosid | 1,00 | 1,00 | **-** |
| Natriumlauroylsarcosinat | - | - | 1,00 |
| Hydroxyethylcellulose | 0,25 | - | 0,25 |
| Xanthangummi | 0,35 | 0,20 | 0,35 |
| Acrylat/C₁₀₋₃₀-Alkylacrylatcrosspolymer | 0,35 | 0,25 | 0,35 |
| PEG-40 Hydriertes Ricinusöl | - | - | - |
| NaOH | 0,15 | 0,12 | 0,15 |
| Glycerin | 5,00 | 2,50 | 5,00 |
| Butylenglycol | - | 2,50 | **-** |
| Panthenol | 1,30 | 1,30 | 1,30 |
| Ethanol | 5,00 | - | 5,00 |
| Parfum, Konservierung, Farbstoffe | q.s. | q.s. | q.s. |
| Wasser | ad 100,00 | ad 100,00 | ad 100,00 |

| **Beispiele** | | | |
|---|---|---|---|
| | 4 | 5 | 6 |
| | Gew.-% | Gew.-% | Gew.-% |
| Natriumlauroylsarcosinat | 1,00 | 1,00 | 1,00 |
| Hydroxyethylcellulose | 0,20 | - | - |
| Xanthangummi | - | 0,20 | 0,35 |
| Acrylat/C₁₀₋₃₀-Alkylacrylatcrosspolymer | 0,30 | 0,20 | 0,25 |
| PEG-40 Hydriertes Ricinusöl | 1,00 | - | - |
| NaOH | 0,15 | 0,10 | 0,12 |
| Glycerin | 2,50 | 2,50 | 2,50 |
| Butylenglycol | 2,50 | 2,50 | 2,50 |
| Panthenol | 1,30 | 1,30 | 1,30 |
| Parfum, Konservierung, Farbstoffe | q.s. | q.s. | q.s. |
| Wasser | ad 100,00 | ad 100,00 | ad 100,00 |

| **Beispiele** | | | |
|---|---|---|---|
| | 7 | 8 | 9 |
| Produktbezeichnung | Gew.-% | Gew.-% | Gew.-% |
| Natriumlauroylsarcosinat | 1,00 | 1,00 | 1,00 |
| Xanthangummi | 0,35 | - | 0,30 |
| Acrylat/C₁₀₋₃₀-Alkylacrylatcrosspolymer | 0,25 | 0,32 | 0,20 |
| PEG-40 Hydriertes Ricinusöl | 0,50 | 1,00 | 0,50 |
| NaOH | 0,12 | 0,16 | 0,12 |
| Glycerin | 2,50 | 2,50 | 5,00 |
| Butylenglycol | 2,50 | 2,50 | - |
| Panthenol | 1,30 | 1,30 | 1,30 |
| Parfum, Konservierung, Farbstoffe | q.s. | q.s. | q.s. |
| Wasser | ad 100,00 | ad 100,00 | ad 100,00 |

Die Zubereitungen gemäß den Beispielen 1 - 9 werden in üblicher Weise bei 25 °C zusammengegeben und verrührt bis eine homogene Masse entstanden ist

## Patentansprüche

1. Verwendung von Zubereitungen, **gekennzeichnet durch** einen Gehalt an
(1) einem oder mehreren N-Acylsarkosinaten
(2) einem oder mehreren Polysacchariden
(3) einer oder mehreren Substanzen, welche in Wasser ein Gelgerüst bilden, welche keine Polysaccharide darstellen und welche vorteilhafterweise grenzflächenaktive Eigenschaften aufweisen
(4) einem oder mehreren Lösungsvermittlern, bevorzugt gewählt aus der Gruppe der polyethoxylierten gesättigten Fett- oder Ölkomponenten
(5) Wasser,
als Hilfsmittel bei der Elektrorasur.

2. Verwendung nach Anspruch 1, **dadurch gekennzeichnet, dass** als N-Acylsarkosinat das Natriumlauroylsarkosinat gewählt wird.

3. Verwendung nach Anspruch 1, **dadurch gekennzeichnet, dass** die Zubereitungen 0,01 bis 10 Gew.%, besonders vorteilhaft 0,01 bis 5 Gew.-%, ganz besonders bevorzugt 0.5 bis 3 Gew.-% an einem oder mehreren einem oder mehreren N-Acylsarkosinaten enthalten, jeweils bezogen auf das Gesamtgewicht der Zubereitungen.

4. Verwendung nach Anspruch 1, **dadurch gekennzeichnet, dass** als Polysaccharid Xanthangummi gewählt wird.

5. Verwendung nach Anspruch 1, **dadurch gekennzeichnet, dass** die Zubereitungen 0,01 bis 10 Gew.-%, besonders vorteilhaft 0,01 bis 5 Gew.-%, ganz besonders bevorzugt 0,5 bis 3 Gew.-% an einem oder mehreren einem oder mehreren Polysacchariden enthalten, jeweils bezogen auf das Gesamtgewicht der Zubereitungen.

6. Verwendung nach Anspruch 1, **dadurch gekennzeichnet, dass** als Substanz, welche in Wasser ein Gelgerüst bildet und welche kein Polysaccharid darstellt, das Carbomer 1342 gewählt wird.

7. Verwendung nach Anspruch 1, **dadurch gekennzeichnet, dass** die Zubereitungen 0,01 bis 10 Gew.-%, besonders vorteilhaft 0,01 bis 5 Gew.-%, ganz besonders bevorzugt 0,5 bis 3 Gew.-% an einer oder mehreren Substanzen enthalten, welche in Wasser ein Gelgerüst bilden und welche keine Polysaccharide darstellen, jeweils bezogen auf das Gesamtgewicht der Zubereitungen,

8. Verwendung nach Anspruch 1, **dadurch gekennzeichnet, dass** als Lösungsvermittler PEG-40-hydriertes Ricinusöl gewählt wird.

9. Verwendung nach Anspruch 1, **dadurch gekennzeichnet, dass** die Zubereitungen 0,01 bis 10 Gew.-%, besonders vorteilhaft 0.01 bis 5 Gew.-%, ganz besonders bevorzugt 0,5 bis 3 Gew.-% an einem oder mehreren Lösungsvermittlern, jeweils bezogen auf das Gesamtgewicht der Zubereitungen.

## Claims

1. Use of preparations, **characterized by** a content of
(1) one or more N-acyl sarcosinates
(2) one or more polysaccharides
(3) one or more substances which form a gel structure in water, which do not constitute polysaccharides and which advantageously have interface-active properties
(4) one or more solubility promoters, preferably selected from the group of polyethoxylated saturated fat or oil components
(5) water,
as auxiliaries in electric shaving.

2. Use according to Claim 1, **characterized in that** sodium lauryl sarcosinate is selected as N-acyl sarcosinate.

3. Use according to Claim 1, **characterized in that** the preparations comprise 0.01 to 10% by weight, particularly advantageously 0.01 to 5% by weight, very particularly preferably 0.5 to 3% by weight, of one or more N-acyl sarcosinates, in each case based on the total weight of the preparations.

4. Use according to Claim 1, **characterized in that** xanthan gum is selected as polysaccharide.

5. Use according to Claim 1, **characterized in that** the preparations comprise 0.01 to 10% by weight, particularly advantageously 0.01 to 5% by weight, very particularly preferably 0.5 to 3% by weight, of one or more polysaccharides, in each case based on the total weight of the preparations.

6. Use according to Claim 1, **characterized in that** Carbomer 1342 is selected as substance which forms a gel structure in water and which does not constitute a polysaccharide.

7. Use according to Claim 1, **characterized in that** the preparations comprise 0.01 to 10% by weight, particularly advantageously 0.01 to 5% by weight, very particularly preferably 0.5 to 3% by weight, of one or more substances which form a gel structure in water and which do not constitute polysaccharides, in each case based on the total weight of the preparations.

8. Use according to Claim 1, **characterized in that** PEG-40 hydrogenated castor oil is selected as solubility promoter.

9. Use according to Claim 1, **characterized in that** the preparations comprise 0.01 to 10% by weight, particularly advantageously 0.01 to 5% by weight, very particularly preferably 0.5 to 3% by weight, of one or more solubility promoters, in each case based on the total weight of the preparations.

## Revendications

1. Utilisation de préparations, **caractérisée par** une teneur en
(1) un ou plusieurs N-acylsarcosinates
(2) un ou plusieurs polysaccharides
(3) une ou plusieurs substances qui forment une structure de gel dans l'eau, qui ne sont pas des polysaccharides et qui présentent avantageusement des propriétés tensioactives,
(4) un ou plusieurs promoteurs de solubilité, de préférence choisis parmi le groupe constitué de composants gras ou huileux saturés polyéthoxylés,
(5) de l'eau,
en tant qu'auxiliaires pour le rasage électrique.

2. Utilisation selon la revendication 1, **caractérisée en ce que** le laurylsarcosinate de sodium est choisi en tant que N-acylsarcosinate.

3. Utilisation selon la revendication 1, **caractérisée en ce que** les préparations comprennent de 0,01 à 10 % en poids, particulièrement avantageusement de 0,01 à 5 % en poids, tout particulièrement préférablement de 0,5 à 3 % en poids d'un ou de plusieurs N-acylsarcosinates, dans chaque cas par rapport au poids total des préparations.

4. Utilisation selon la revendication 1, **caractérisée en ce que** la gomme de xanthane est choisie en tant que polysaccharide.

5. Utilisation selon la revendication 1, **caractérisée en ce que** les préparations comprennent de 0,01 à 10 % en poids, particulièrement avantageusement de 0,01 à 5 % en poids, tout particulièrement avantageusement de 0,5 à 3 % en poids d'un ou de plusieurs polysaccharides, dans chaque cas par rapport au poids total des préparations.

6. Utilisation selon la revendication 1, **caractérisée en ce que** le Carbomer 1342 est choisi en tant que substance qui forme une structure de gel dans l'eau et qui n'est pas un polysaccharide.

7. Utilisation selon la revendication 1, **caractérisée en ce que** les préparations comprennent de 0,01 à 10 % en poids, particulièrement avantageusement de 0,01 à 5 % en poids, tout particulièrement avantageusement de 0,5 à 3 % en poids d'une ou de plusieurs substances qui forment une structure de gel dans l'eau et ne sont pas des polysaccharides, dans chaque cas par rapport au poids total des préparations.

8. Utilisation selon la revendication 1, **caractérisée en ce que** l'huile de ricin hydrogénée de PEG-40 est choisie en tant que promoteur de solubilité.

9. Utilisation selon la revendication 1, **caractérisée en ce que** les préparations comprennent de 0,01 à 10 % en poids, particulièrement avantageusement de 0,01 à 5 % en poids, tout particulièrement avantageusement de 0,5 à 3 % en poids d'un ou de plusieurs promoteurs de solubilité, dans chaque cas par rapport au poids total des préparations.
